# EUROPEAN PATENT SPECIFICATION

(11) **EP 1 482 899 B1**
(45) Date of publication and mention of the grant of the patent: **17.05.2006**
(21) Application number: 03717184.0
(22) Date of filing: 12.02.2003
(51) Int. Cl.: A61K 8/06, A61K 8/895, A61Q 5/12

(54) **HAIR TREATMENT COMPOSITIONS**
HAARBEHANDLUNGSZUSAMMENSETZUNGEN
COMPOSITIONS DE SOIN CAPILLAIRE

(30) Priority: 08.03.2002 GB 0205531
(43) Date of publication of application: 08.12.2004
(73) Proprietor: UNILEVER PLC, London EC4P 4BQ (GB); UNILEVER N.V., 3013 AL Rotterdam (NL)
(72) Inventor: MAHADESHWAR, Anand R., Unilever R&D Port Sunlight, Wirral, Merseyside CH63 3JW (GB); TAN-WALKER, Ruby L. B., Unilever R&D Port Sunlight, Wirral, Merseyside CH63 3JW (GB); VEIRO, Jeffrey A., Lever Pond's (Pty) Ltd, 4051 La Lucia Ridge Estate (ZA)
(74) Representative: Tansley, Sally Elizabeth
(86) International application number: PCT/EP2003/001399
(87) International publication number: WO 2003/075866

(56) References cited:
- WO-A-93/08787
- WO-A-97/33559
- US-A- 5 084 208
- US-A- 5 415 857

## Description

### Background to the Invention

Shampoo compositions which provide a combination of cleansing and conditioning to the hair are known in the prior art. Such shampoo compositions typically comprise one or more surfactants for shampooing or cleansing purposes and one or more conditioning agents. The purpose of the conditioning agent is to make the hair easier to comb when wet and more manageable when dry, e.g. less static and fly-away. Typically, these conditioning agents are either water-insoluble oily materials or cationic materials.

Amongst the most popular conditioning agents used in shampoo products are silicone polymers, present in the shampoo as emulsion droplets. Conditioning is achieved by the silicone being deposited onto the hair resulting in the formation of a film. Whilst the silicone film gives excellent conditioning, for example wet comb properties, the repeated use of compositions containing silicones can lead to a build-up of silicone and undesirable affects such as a heavy, oily feel to the hair. This can lead to the perception by some users that their hair is not clean.

A problem associated with use of cationic polymers and cationic surfactants as conditioning agents is that the anionic surfactants commonly employed in shampoo compositions as cleansing surfactants can interact with the cationic conditioning agents resulting in poorer performance of the conditioning agent and/or the cleansing surfactant.

Attempts to overcome this problem have been made by employing nonionic, amphoteric and/or cationic co-surfactants as cleansing agents, but these systems do not generally provide as high a level of cleansing or foaming as do surfactant systems comprising anionic surfactant.

Many consumers habitually apply triglycerides to their hair as part of their regular grooming habits. Coconut oil is frequently used as a grooming aid by such consumers. Silicones in hair treatment formulations are not generally compatible with film formation on oiled hair to provide conditioning benefits. There is a need for hair treatment compositions which can provide conditioning benefits to oiled hair.

Another class of conditioning agents used in hair treatment compositions is non-silicone water-insoluble oily materials. Such oily materials have been used to a far lesser degree than silicone and cationic conditioning agents. One reason for this is the incompatibility of such materials with hair treatment surfactants, especially anionic surfactants used in shampoos to provide good cleaning. Another reason is the inherent instability of emulsions of these oily materials in aqueous-based hair treatment compositions. This often results in a lack of homogeneity in the hair treatment composition and/or inadequate shelf-life.

Accordingly, there is a need for hair treatment compositions containing reduced levels of silicone oily conditioning agents to prevent build-up yet which deliver both good conditioning performance, and which are also stable in the hair treatment composition. There is also a need for hair treatment compositions which are effective for conditioning the hair of consumers who regularly apply triglycerides to their hair. There is also a need for hair treatment compositions which deliver conditioning benefits from a cleansing composition without making the users feel that their hair is not clean after use.

EP 0 742 64 discloses aqueous conditioning shampoos comprising anionic or amphoteric surfactant, cationic or nonionic conditioning agents, and a water miscible saccharide. Suitable conditioning agents include silicones, resinous materials, waxy materials and oily materials.

WO 93/08787 discloses conditioning shampoos including anionic surfactant, dispersed non-volatile nonionic silicone conditioning agent, a water-soluble cationic organic hair-conditioning polymer of specified charge density and an organic, non-volatile, water-insoluble liquid selected from hydrocarbon oils, fatty esters and mixtures thereof.

US 5,344,643 discloses shampoo compositions comprising an anionic surfactant, an oily, water-insoluble conditioning agent, a carboxyvinyl polymer suspending and stabilising agent for the oily conditioning agent and a cationic conditioning agent.

FR 2788971 discloses a hair treatment composition containing an oil with at least one amine and one urethane group and a conditioner which may be polyolefin, cationic or amphoteric polymers, cationic proteins and their hydrolysates, silicones, mineral oils, ceramide-type compounds or cationic surfactants.

WO 97/33559 discloses cosmetic compositions comprising microdroplets of silicone and mineral oil blends.

US 5,084,208 provides cosmetic formulations containing discrete microdroplets of a cosmetically active oil including silicon oil. Alkyl functionalised silicone are used.

US 5,415,857 discloses hair conditioning compositions comprising aminosilicone and hydrocarbons.

It has now found that by the use of discrete droplets containing both mineral oil and functionalised silicone, pre-emulsified before its addition to the hair treatment composition, a composition can be provided which supplies conditioning benefits to the hair of hair oil users. Compositions according to the invention can also be beneficial in reducing the long-term build up of silicones on the hair. Compositions according to the invention can also provide conditioning benefits from a cleansing product with good stability.

### Summary of the Invention

According to one aspect of the invention, there is provided a composition for hair treatment comprising discrete droplets characterised in that the droplets, within the same droplet comprise both;
(i) a functionalised silicone selected from amino-, carboxy-, betaine-, quaternary ammonium-, carbohydrate-, hydroxy- and alkoxy-substituted silicone and
(ii) a hydrocarbon oil;
wherein the discrete droplets comprise at least 5% by weight of functionalised silicone and at least 5% by weight of hydrocarbon oil expressed as a percentage of the weight of the droplets.

### Detailed Description of the Invention

By "water-insoluble" is meant that a material is not soluble in water at a concentration of 0.5% by weight, based on weight of water, at 25°C.

Where values for viscosity are specified, these refer to kinematic viscosities measured using suitable apparatus at 25°C. A suitable apparatus is a capillary viscometer.

As used herein, the term "mixture droplet" is used to refer to the discrete droplets comprising both hydrocarbon oil and functionalised silicone.

By discrete droplets, it is meant that whereas the main body of the composition is water-continuous, the droplets form a separate, discontinuous, non-aqueous phase.

It is essential to the invention that both hydrocarbon oil and functionalised silicone are present within common droplets. In other words, within a single droplet, both hydrocarbon oil and functionalised silicone must be present together. However, it is not essential to the invention that every non-aqueous droplet within the composition has this form, so long as an effective amount of the mixture droplets is present in the composition.

In a preferred form of a composition according to the invention, the composition comprises at 0.25 to 0.5% by weight of the mixture droplets.

The mixture droplets comprises at least 5% by weight of functional silicone and at least 5% by weight of hydrocarbon oil expressed as a percentage of the weight of the mixture droplets.

### Functionalised Silicone

The functionalised silicone is present in the mixture droplets at a level of at least 5 percent by weight, preferably at least 25 percent by weight, more preferably at least 40 percent by weight and most preferably at least 50% by weight based on the total weight of the mixture droplets.

Suitably, the functionalised silicone has a kinematic viscosity of less than 1000 mm²s⁻¹, preferably less than 500 mm²s⁻¹, more preferably less than 200 mm²s⁻¹ at 25°C.

The functionalised silicones include, amino-, carboxy-, betaine-, quaternary ammonium-, carbohydrate-, hydroxy- and alkoxy-substituted silicones.

Preferably, the functionalised silicone contains multiple substitutions.

By functionalised silicone is meant a polymer or oligomer with a backbone consisting essentially of polydialkylsiloxane monomers, preferably polydimethylsiloxane monomers, wherein the alkyl groups have been partially substituted by functional organic groups.

For the avoidance of doubt, as regards hydroxyl-substituted silicones, a polydimethylsiloxane merely having hydroxyl end groups (which have the CTFA designation dimethiconol) is not considered a functionalised silicone within the present invention. However, a polydimethylsiloxane having hydroxyl substitutions along the polymer chain is considered a functionalised silicone.

Preferred functionalised silicones are amino-functionalised silicones. Suitable amino functionalised silicones are described in EP 455,185 (Helene Curtis) and include trimethylsilylamodimethicone as depicted below:

Si(CH₃)₃ - O - [Si(CH₃)₂ - O -]ₓ - [Si (CH₃) (R - NH - R₁ NH₂) - O -]_{y} - Si (CH₃)₃

wherein x + y is a number from about 50 to about 500 and wherein R is an alkylene group having from 2 to 5 carbon atoms and R₁ is a second alkylene group having from 2 to 4 carbon atoms. Preferably, the number x + y is in the range of from about 100 to about 300.

As expressed here, the weight percent amine functionality is measured by titrating a sample of the amino-functionalised silicone against alcoholic hydrochloric acid to the bromocresol green end point. The weight % amine is calculated using a molecular weight of 45 (corresponding to CH₃- CH₂-NH₂).

Suitably, the weight percent amine functionality measured and calculated in this way is from 0.3% to 8%, preferably from 0.5% to 4%.

An example of a commercially available amino-functionalised silicone useful in the silicone component of the composition of the invention is DC8220 available from Dow Corning, which has a viscosity of 150 mm²s⁻¹ at 25°C and a weight percent amine functionality of 2.0%.

### Hydrocarbon oil

The hydrocarbon oil is present in the mixture droplets at a level of at least 5 percent by weight, preferably at least 15 percent by weight more preferably at least 20 percent by weight and most preferably at least 30% by weight based on the total weight of the mixture droplets.

Suitably, the hydrocarbon oil has a kinematic viscosity at 25°C of less than 500 mm²s⁻¹ , preferably less than 300 mm²s⁻¹, more preferably less than 200 mm²s⁻¹ and most preferably less than 50 mm²s⁻¹.

Hydrocarbon oils include cyclic hydrocarbons, straight chain aliphatic hydrocarbons (saturated or unsaturated), and branched chain aliphatic hydrocarbons (saturated or unsaturated). The hydrocarbon oils will preferably contain from 12 to 60 carbon atoms. Also suitable are polymeric hydrocarbons of alkenyl monomers, such as C₂-C₆ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene. A commercially available material of this type is L-14 polybutene from Amoco Chemical Co. (Chicago, Ill., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons ranging from C₁₆H₃₄ to C₂₁H₄₄. Suitable commercially available materials of this type include Sirius M85 and Sirius M45, available from Silkolene (Trademark).

### Mixture Droplets

In compositions according to the invention, the mixture droplets form a separate, discontinuous phase from the continuous phase of the hair treatment composition. Generally, the continuous phase of the hair treatment composition will be water-continuous.

In order for the mixture droplets to remain stable as discrete droplets over time, it is preferred if the functionalised silicone and the hydrocarbon oil are insoluble in the continuous phase of the hair treatment composition.

In a preferred form of the invention, the continuous phase of the hair treatment composition will be water-based and the hydrocarbon oil and functionalised silicone will be water-insoluble. In this case, the mixture droplets will be dispersed in the composition as the oil droplets of an oil-in-water emulsion.

The mixture droplets may be liquid, semi-solid or solid in nature, so long as they are substantially uniformly dispersed in the fully formulated product. The mixture droplets are preferably present as either liquid or semi-solid droplets, more preferably as liquid droplets.

The D_{3,2} average droplet size of the mixture droplets is preferably greater than 0.05 micrometre, more preferably at least 0.5 micrometre, most preferably at least 1 micrometre.

Additionally, the D_{3,2} average droplet size of the mixture droplets is no greater than 25 micrometres, preferably no greater than 20 micrometres, more preferably no greater than 15 micrometres. It should be noted that suitable droplet size ranges include any maximum D_{3,2} average droplet size associated with any minimum D_{3,2} average droplet size. A preferred range is from 0.05 to 25 micrometres, more preferably from 0.5 to 20 micrometres, most preferably from 1 to 15 micrometres.

Methods for measuring the D_{3,2} average droplet size are well known to those skilled in the art, and may require dilution of the composition prior to measurement. One suitable method is by means of a laser light scattering technique, using a 2600D Particle Sizer from Malvern Instruments.

It is a highly preferred aspect of the invention that the mixture droplets be in the form of the discontinuous phase of an aqueous oil-in-water emulsion which may itself be added to the hair treatment composition during manufacture. Preferably, the aqueous emulsion is mechanically-formed. In such emulsions, it is preferable that the emulsion additionally includes at least one emulsifier in order to stabilise the emulsion.

Another aspect of the invention is a method for incorporating discrete droplets comprising both a functionalised silicone selected from amino-, carboxy-, betaine-, quaternary ammonium-, carbohydrate-, hydroxy,- and alkoxy-substituted silicones; and a hydrocarbon oil in the same droplets, into a hair treatment composition, comprising the steps of;
i) forming an intimate, non-aqueous blend comprising the functionalised silicone and the hydrocarbon oil,
ii) preparing an aqueous emulsion comprising droplets comprising both a functionalised silicone and a hydrocarbon oil in the same droplets and
i) mixing said aqueous emulsion with the hair treatment composition.

Suitable emulsifiers for use in the preparation of the aqueous emulsion are well known in the art and include anionic, cationic, zwitterionic, amphoteric and nonionic surfactants, and mixtures thereof. Examples of anionic surfactants used as emulsifiers for the silicone particles are alkylarylsulphonates, e.g., sodium dodecylbenzene sulphonate, alkyl sulphates e.g., sodium lauryl sulphate, alkyl ether sulphates, e.g., sodium lauryl ether sulphate nEO, where n is from 1 to 20 alkylphenol ether sulphates, e.g., octylphenol ether sulphate nEO where n is from 1 to 20, and sulphosuccinates, e.g., sodium dioctylsulphosuccinate.

Examples of nonionic surfactants used as emulsifiers for the silicone particles are alkylphenol ethoxylates, e.g., nonylphenol ethoxylate nEO, where n is from 1 to 50 and alcohol ethoxylates, e.g., lauryl alcohol nEO, where n is from 1 to 50, ester ethoxylates, e.g., polyoxyethylene monostearate where the number of oxyethylene units is from 1 to 30.

It is preferred if the emulsifier is blended into the oil phase prior to the formation of the aqueous emulsion of the mixture droplets.

A preferred process for preparing aqueous emulsions of the mixture droplets which can then be incorporated in the hair treatment compositions involves use of a high-shear mixer. Suitable mixers should be capable of handling high viscosity liquids at low temperatures. Preferably, the mixer is a hollow cylinder or bowl-shaped and comprises a centrally-mounted, rotatable shaft carrying thereon tools or blades which rotate with the shaft.

Suitably, the clearance of the tips of the tools or blades from the wall of the mixer is relatively small, e.g. less than 20 mm, preferably less than 15 mm, more preferably less than 10 mm. The speed of rotation of the shaft will vary depending on the dimensions of the mixer but will typically be in the region of 100-1200 rpm.

Preferably, the mixer is also capable of having the temperature of mixing controlled, e.g. it comprises a jacket through which a heat transfer fluid can be circulated.

In order to obtain an intimate blend of the functionalised silicone and the hydrocarbon oil in the mixture droplets, it is highly preferred that both components should be liquid at the temperature at which mixing and emulsification takes place.

### Hair Treatment Compositions

Hair treatment compositions according to the invention may suitably take the form of shampoos, conditioners, sprays, mousses or lotions. Preferred hair treatment composition forms are shampoos, conditioners and mousses.

Another aspect of the invention is the use of hair treatment compositions according to the invention for the conditioning of hair. A preferred use of compositions according to the invention is as a conditioning shampoo.

### Shampoo Compositions

A preferred hair treatment composition in accordance with the invention is a conditioning shampoo composition. Such a shampoo composition will comprise one or more cleansing surfactants which are cosmetically acceptable and suitable for topical application to the hair. Further surfactants may be present as an additional ingredient if sufficient for cleansing purposes is not provided by the emulsifier for the mixture droplets. It is preferred that shampoo compositions according to the invention comprise at least one further surfactant (in addition to that used as emulsifying agent for the conditioning mixture) to provide a cleansing benefit.

Suitable cleansing surfactants, which may be used singularly or in combination, are selected from anionic, cationic, nonionic, amphoteric and zwitterionic surfactants and mixtures thereof. The cleansing surfactant may be the same surfactant as the emulsifier, or may be different.

Shampoo compositions according to the invention will typically comprise one or more anionic cleansing surfactants that are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate (n) EO, (where n ranges from 1 to 3).

Mixtures of any of the foregoing anionic cleansing surfactants may also be suitable.

The total amount of anionic cleansing surfactant in shampoo compositions of the invention is generally from 5 to 30, preferably from 6 to 20, more preferably from 8 to 18 wt%.

The shampoo composition can optionally include co-surfactants, to help impart aesthetic, physical or cleansing properties to the composition.

A preferred example is an amphoteric or zwitterionic surfactant, which can be included in an amount ranging from 0 to about 8, preferably from 1 to 4 percent by weight of the composition.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred example is a nonionic surfactant, which can be included in an amount ranging from 0.1 to 8, preferably from 1 to 5 percent by weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition may also optionally include one or more cationic co-surfactants included in an amount ranging from 0.01 to 10, more preferably from 0.02 to 5, most preferably from 0.025 to 2 percent by weight of the composition.

The total amount of surfactant (including any co-surfactant, and/or any emulsifier for the silicone component) in shampoo compositions of the invention is generally from 0.1 to 50, preferably from 5 to 30, more preferably from 10 to 25 percent by weight of the composition.

### Cationic Deposition Polymer

A cationic deposition polymer is an optional ingredient in shampoos according to the invention, for enhancing conditioning performance of the compositions. By "deposition polymer" is meant an agent which enhances deposition of the mixture droplets from the hair treatment composition onto the intended site during use, i.e. the hair and/or the scalp.

The deposition polymer may be a homopolymer or be formed from two or more types of monomers. The molecular weight of the polymer will generally be between 5 000 and 10 000 000, typically at least 10 000 and preferably in the range 100 000 to about 2 000 000 unified atomic mass units. The polymers will have cationic nitrogen containing groups such as quaternary ammonium or protonated amino groups or a mixture thereof.

The cationic nitrogen-containing group will generally be present as a substituent on a fraction of the total monomer units of the deposition polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic deposition polymers can comprise mixtures of monomer units derived from amine- and/or quaternary ammonium-substituted monomer and/or compatible spacer monomers.

Suitable cationic deposition polymers include, for example:
- copolymers of 1-vinyl-2-pyrrolidine and 1-vinyl-3-methylimidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311).

Other cationic deposition polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g.

Cationic polysaccharide polymers suitable for use in compositions of the invention include those of the formula:

A-O-[R-N⁺(R¹) (R²) (R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (Commercially available from Rhone-Poulenc in their JAGUAR trademark series) .

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic deposition polymer is selected from cationic cellulose and cationic guar derivatives.

Particularly preferred deposition polymers are JAGUAR C13S, JAGUAR C15, JAGUAR C17 and JAGUAR C16 and JAGUAR C162.

### Conditioner Compositions

Compositions in accordance with the invention may also be formulated as conditioners for the treatment of hair, typically applied to the hair after shampooing and subsequent rinsing.

### Conditioning Surfactant

Such a conditioner will comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

In conditioners of the invention, the level of cationic surfactant is preferably from 0.01 to 10, more preferably 0.05 to 5, most preferably 0.1 to 2 percent by weight of the total composition.

### Fatty Alcohol

Conditioners according to the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10, preferably from 0.1 to 5 percent by weight of the total composition. The weight ratio of cationic surfactant to fatty alcohol is suitably from 10:1 to 1:10, preferably from 4:1 to 1:8, optimally from 1:1 to 1:4.

### Mousses

Hair treatment compositions in accordance with the invention may also take the form of aerosol foams (mousses) in which case a propellant must be included in the composition. This agent is responsible for expelling the other materials from the container and forming the hair mousse character.

The propellant gas can be any liquefiable gas conventionally used for aerosol containers. Examples of suitable propellants include dimethyl ether, propane, n-butane and isobutane, used singly or in admixture.

The amount of the propellant gases is governed by normal factors well known in the aerosol art. For hair mousses, the level of propellant is generally from 3 to 30, preferably from 5 to 15 percent by weight of the total composition.

Small quantities of surfactant ranging anywhere from 0.1 to 10, preferably from 0.1 to about 1, for example 0.3 percent by weight of the composition may be present in the hair mousse compositions of the invention. The surfactant may be an anionic, nonionic or cationic emulsifier. Particularly preferred are nonionic emulsifiers which are formed from alkoxylation of hydrophobes such as fatty alcohols, fatty acids and phenols.

### Optional Ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, antimicrobials and sunscreens. Each of these ingredients will be present in an amount effective to accomplish its purpose. Generally these optional ingredients are included individually at a level of up to 5 percent by weight of the total composition.

Preferably, compositions of this invention also contain adjuvants suitable for hair care. Generally such ingredients are included individually at a level of up to 2, preferably up to 1 percent by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.

### Mode of Use

The compositions of the invention are primarily intended for topical application to the hair and/or scalp of a human subject to improve hair fibre surface properties such as smoothness, softness, manageability, cuticle integrity, and shine.

The invention will now be further illustrated by the following, non-limiting examples:

### Examples

Shampoo formulations were made as shown in table 1. Examples land 2 are comparative examples, whereas examples A and B are shampoo compositions according to the invention. All figures are weight percent of the compositions.

**Table 1**

| **Ingredient** | **Trade Name** | **Supplier** | **Ex.1** | **Ex.2** | **Ex.B** | **Ex.A** |
|---|---|---|---|---|---|---|
| Sodium laureth (2 EO) sulphate | Empicol ESB70 | Albright & Wilson | 16 | 16 | 16 | 16 |
| Coco amidopropyl betaine | Tegobetaine CK | Goldschmidt | 2 | 2 | 2 | 2 |
| Guar Hydroxypropyl Trimonium Chloride | Jaguar C13S | Rhone Poulenc | 0.1 | 0.1 | 0.1 | 0.1 |
| Mineral Oil | Mineral oil M40 | Fuchs Lubricants | 1 | - | - | - |
| Amino silicone | DC8220 | Dow Corning | - | 1 | - | - |
| Pre-emulsified blend of 0.5 Mineral oil+0.5 Amino silicone DC8466 | - | - | - | - | 1 | - |
| Pre-emulsified blend of 0.5 Mineral oil+ 0.5 Amino silicone DC8220 | - | - | - - | - | - | 1 |
| Water | - | - | to 100 | to 100 | to 100 | to 100 |

The aminosilicone DC8220 has a viscosity of 150 mm²s⁻¹ at 25°C and a weight percent amine functionality of 2.0%. The aminosilicone DC8466 has a viscosity of 15000 mm²s⁻¹ at 25°C and a weight percent amine functionality of 2.3%.

Mineral oil M4 has a viscosity of 4.3 mm²s⁻¹ at 25°C.

### Test Method

Hair switches were oiled with commercially available oil, which is a blend of 60% coconut oil and 40% mineral oil (M40). 0.5ml of oil was applied on to the switches and the switches were allowed to stay for an hour. 0.35ml of test formulation was measured and applied onto the oiled hair switches, followed by washing and rinsing in accordance with normal procedures. The shampooing and rinsing procedure was repeated for second application and then the switches were allowed to dry at normal temperature (20-25°C). On drying, the switches were assessed by panellists with previous experience in assessing hair switch characteristics.

### Evaluation results

The rating scores are shown in the Tables below for the attributes described. Higher scores indicate better results.

**Table 2**

| **Attribute** | **Ex.1** | **Ex.A** |
|---|---|---|
| Ease of Comb | 0.28 | 0.53 |
| Clean feel | 0.29 | 0.38 |

**Table 3**

| **Attribute** | **Ex.2** | **Ex.A** |
|---|---|---|
| Ease of Comb | 0.19 | 0.53 |
| Clean feel | 0.33 | 0.38 |

**Table 4**

| **Attribute** | **Ex.B** | **Ex.A** |
|---|---|---|
| Ease of Comb | 0.25 | 0.3 |
| Clean feel | 0.25 | 0.3 |

The results show that the example according to the invention (A) delivers improved conditioning relative to the comparative examples 1 and 2 without affecting the clean feel.

Table 4 shows that example A, with a lower viscosity functionalised silicone, is preferred over example B with a higher viscosity functionalised silicone.

## Claims

1. A composition for hair treatment comprising discrete droplets **characterised in that** the droplets, within the same droplet comprise both:
(i) a functionalised silicone selected from amino-, carboxy-, betaine-, quaternary ammonium-, carbohydrate-, hydroxy- and alkoxy-substituted silicones; and,
(ii) a hydrocarbon oil;
wherein the discrete droplets comprise at least 5% by weight of functionalised silicone and at least 5% by weight of hydrocarbon oil expressed as a percentage of the weight of the droplets.

2. A composition according to claim 1 comprising at least 0.25% by weight of the discrete droplets.

3. A composition according to any preceding claim, wherein the functionalised silicone is an amino-functionalised silicone.

4. A composition according to claim 3 wherein the amino-functionalised silicone has a weight percentage amine functionality from 0.3% to 8%, preferably from 0.5% to 4%.

5. A composition according to any preceding claim wherein the hydrocarbon oil has a kinematic viscosity of less than 500 mm²s⁻¹ at 25°C.

6. A composition according to any preceding claim wherein the kinematic viscosity of the functionalised silicone is less than 1000 mm²s⁻¹ at 25°C.

7. A composition according to any preceding claim wherein the mean droplet diameter (D_{3,2}) of the discrete droplets is from 0.05 to 25 micrometres.

8. A composition according to any preceding claim wherein the discrete droplets further comprise an emulsifying agent.

9. A composition according to claim 8 wherein the emulsifying agent comprises a nonionic surfactant.

10. A composition according to any preceding claim wherein the discrete droplets are added to the composition as a non-aqueous phase of a pre-formed aqueous emulsion.

11. A composition according to any preceding claim which is a shampoo composition comprising at least one cleansing surfactant selected from anionic, cationic, nonionic, amphoteric and zwitterionic surfactants and mixtures thereof.

12. A composition according to any one of claims 1 to 10, which is a conditioner composition comprising at least one conditioning surfactant and a fatty alcohol and/or an alkoxylated fatty alcohol.

13. A method for incorporating discrete droplets comprising both a functionalised silicone selected from amino-, carboxy-, betaine-, quaternary ammonium-, carbohydrate-, hydroxy- and alkoxy-substituted silicones; and a hydrocarbon oil in the same droplets, into a hair treatment composition, comprising the steps of:
i) forming an intimate, non-aqueous blend comprising the functionalised silicone and the hydrocarbon oil,
ii) preparing an aqueous emulsion comprising droplets comprising both a functionalised silicone and a hydrocarbon oil in the same droplets and
iii) mixing said aqueous emulsion with the hair treatment composition.

14. A method according to claim 13 wherein the aqueous emulsion further comprises an emulsifying agent.

15. The use of a hair treatment composition according to any one of claims 1 to 11 for the conditioning of hair.

16. The use of a hair treatment composition according to any one of claims 1 to 11 as a conditioning shampoo.

## Patentansprüche

1. Zusammensetzung zur Haarbehandlung, umfassend einzelne Tröpfchen, **dadurch gekennzeichnet, daß** die Tröpfchen in demselben Tröpfchen sowohl:
(i) ein funktionalisiertes Silikon, ausgewählt aus Amino-, Carboxy-, Betain-, Quartärammonium-, Kohlenhydrat-, Hydroxy- und Alkoxy-substituierten Silikonen, und
(ii) ein Kohlenwasserstofföl
umfassen;
wobei die einzelnen Tröpfchen mindestens 5 Gew.-% funktionalisiertes Silikon und mindestens 5 Gew.-% Kohlenwasserstofföl, ausgedrückt als ein Prozentsatz des Gewichtes der Tröpfchen, umfassen.

2. Zusammensetzung nach Anspruch 1, umfassend mindestens 0,25 Gew.-% der einzelnen Tröpfchen.

3. Zusammensetzung nach einem der vorhergehenden Ansprüche, wobei das funktionalisierte Silikon ein Amino-funktionalisiertes Silikon ist.

4. Zusammensetzung nach Anspruch 3, wobei das Amino-funktionalisierte Silikon eine Aminfunktionalität in Gewichtsprozent von 0,3 % bis 8 %, vorzugsweise 0,5 % bis 4 % aufweist.

5. Zusammensetzung nach einem der vorhergehenden Ansprüche, wobei das Kohlenwasserstofföl eine kinematische Viskosität von weniger als 500 mm²s⁻¹ bei 25 °C aufweist.

6. Zusammensetzung nach einem der vorhergehenden Ansprüche, wobei die kinematische Viskosität des funktionalisierten Silikons weniger als 1.000 mm²s⁻¹ bei 25 °C beträgt.

7. Zusammensetzung nach einem der vorhergehenden Ansprüche, wobei der mittlere Tröpfchendurchmesser (D_{3,2}) der einzelnen Tröpfchen 0,05 bis 25 µm beträgt.

8. Zusammensetzung nach einem der vorhergehenden Ansprüche, wobei die einzelnen Tröpfchen ferner einen Emulgator umfassen.

9. Zusammensetzung nach Anspruch 8, wobei der Emulgator ein nicht-ionisches oberflächenaktives Mittel umfaßt.

10. Zusammensetzung nach einem der vorhergehenden Ansprüche, wobei die einzelnen Tröpfchen zu der Zusammensetzung als eine nicht-wässerige Phase einer vorgeformten wässerigen Emulsion zugegeben werden.

11. Zusammensetzung nach einem der vorhergehenden Ansprüche, die eine Shampoozusammensetzung ist, umfassend mindestens ein oberflächenaktives Reinigungsmittel, ausgewählt aus anionischen, kationischen, nicht-ionischen, amphoteren und zwitterionischen oberflächenaktiven Mitteln und Gemischen davon.

12. Zusammensetzung nach einem der Ansprüche 1 bis 10, die eine Konditionerzuammensetzung ist, umfassend mindestens ein oberflächenaktives Konditioniermittel und einen Fettalkohol und/oder einen alkoxylierten Fettalkohol.

13. Verfahren zur Einführung einzelner Tröpfchen, die sowohl ein funktionalisiertes Silikon, ausgewählt aus Amino-, Carboxy-, Betain-, Quartärammonium-, Kohlenhydrat-, Hydroxy- und Alkoxy-substituierten Silikonen, als auch ein Kohlenwasserstofföl in den selben Tröpfchen umfassen, in eine Haarbehandlungszusammensetzung, umfassend die Schritte:
i) Bildung eines innigen, nicht wässerigen Gemisches, umfassend das funktionalisierte Silikon und das Kohlenwasserstofföl,
ii) Herstellung einer wässerigen Emulsion, umfassend Tröpfchen, die sowohl ein funktionalisiertes Silikon als auch ein Kohlenwasserstofföl in denselben Tröpfchen umfassen und
iii) Mischen der wässerigen Emulsion mit der Haarbehandlungszusammensetzung.

14. Verfahren nach Anspruch 13, wobei die wässerige Emulsion ferner einen Emulgator umfaßt.

15. Verwendung einer Haarbehandlungszusammensetzung nach einem der Ansprüche 1 bis 11 zur Konditionierung des Haars.

16. Verwendung einer Haarbehandlungszusammensetzung nach einem der Ansprüche 1 bis 11 als Konditioniershampoo.

## Revendications

1. Composition de soin capillaire contenant des gouttelettes discrètes, **caractérisée en ce que** les mêmes gouttelettes contiennent à la fois :
(i) un silicone fonctionnalisé à choisir parmi des silicones amino substitué, carboxy substitué, bétaïne substitué, ammonium quaternaire substitué, hydrate de carboné substitué, hydroxy substitué et alkoxy substitué ; et
(ii) une huile à base d'hydrocarbures ;
dans laquelle les gouttelettes discrètes contiennent au moins 5 % en poids de silicone fonctionnalisé et au moins 5 % en poids d'huile à base d'hydrocarbures, exprimés en pourcentage du poids des gouttelettes.

2. Composition selon la revendication 1 contenant au moins 0,25 % en poids de gouttelettes discrètes.

3. Composition selon l'une quelconque des revendications précédentes, dans laquelle le silicone fonctionnalisé est un silicone amino fonctionnalisé.

4. Composition selon la revendication 3, dans laquelle le silicone amino fonctionnalisé présente un pourcentage en poids de fonctionnalité amine de 0,3 % à 8 %, de préférence de 0,5 % à 4 %.

5. Composition selon l'une quelconque des revendications précédentes, dans laquelle l'huile à base d'hydrocarbures présente une viscosité cinématique inférieure à 500 mm²s⁻¹ à 25°C.

6. Composition selon l'une quelconque des revendications précédentes, dans laquelle la viscosité cinématique du silicone fonctionnalisé est inférieure à 1 000 mm²s⁻¹ à 25°C.

7. Composition selon l'une quelconque des revendications précédentes, dans laquelle le diamètre moyen (D_{3,2}) des gouttelettes discrètes est de 0,05 à 25 micromètres.

8. Composition selon l'une quelconque des revendications précédentes, dans laquelle les gouttelettes discrètes contiennent en outre un agent émulsifiant.

9. Composition selon la revendication 8, dans laquelle l'agent émulsifiant contient un tensioactif non ionique.

10. Composition selon l'une quelconque des revendications précédentes, dans laquelle les gouttelettes discrètes sont ajoutées à la composition en tant que phase non aqueuse d'une émulsion aqueuse formée préalablement.

11. Composition selon l'une quelconque des revendications précédentes, laquelle, est une composition de shampooing comprenant au moins un tensioactif de nettoyage à choisir parmi des tensioactifs anioniques, cationiques, non ioniques, amphotériques et zwitterioniques et des mélanges de ceux-ci.

12. Composition selon l'une quelconque des revendications 1 à 10, laquelle est une composition d'après-shampooing comprenant au moins un tensioactif revitalisant et un alcool gras et/ou un alcool gras alkoxylé.

13. Méthode d'incorporation de gouttelettes discrètes contenant à la fois un silicone fonctionnalisé à choisir parmi des silicones amino substitué, carboxy substitué, bétaïne substitué, ammonium quaternaire substitué, hydrate de carbone substitué, hydroxy substitué et alkoxy substitué et une huile à base d'hydrocarbures dans les mêmes gouttelettes, dans une composition de soin capillaire, comprenant les étapes suivantes :
(i) formation d'un mélange intime non aqueux comprenant le silicone fonctionnalisé et l'huile à base d'hydrocarbures ;
(ii) préparation d'une émulsion aqueuse comportant des gouttelettes comprenant à la fois un silicone fonctionnalisé et une huile à base d'hydrocarbures dans les mêmes gouttelettes ; et
(iii) mélange de ladite émulsion aqueuse avec la composition de soin capillaire.

14. Méthode selon la revendication 13, dans laquelle l'émulsion aqueuse comprend en outre un agent émulsifiant.

15. Utilisation d'une composition de soin capillaire selon l'une quelconque des revendications 1 à 11 pour la revitalisation des cheveux.

16. Utilisation d'une composition de soin capillaire selon l'une quelconque des revendications 1 à 11 comme shampoing revitalisant.
